# FASCICULE DE BREVET EUROPEEN

(11) **EP 2 214 689 B1**
(45) Date de publication et mention de la délivrance du brevet: **17.10.2018**
(21) Numéro de dépôt: 08856862.1
(22) Date de dépôt: 28.11.2008
(51) Int. Cl.: A61K 36/898, A61P 17/00, A61K 8/97, A61P 29/00, A61Q 19/08

(54) **COMPOSITION COSMETIQUE OU DERMATOLOGIQUE CONTENANT UN EXTRAIT D'ORCHIDEE ET METHODE DE SOIN COSMETIQUE UTILISANT CETTE COMPOSITION.**
KOSMETISCHE ODER DERMATOLOGISCHE ZUSAMMENSETZUNG MIT EINEM ORCHIDEENEXTRAKT UND KOSMETISCHES PFLEGEVERFAHREN UNTER VERWENDUNG BESAGTER ZUSAMMENSETZUNG
COSMETIC OR DERMATOLOGICAL COMPOSITION CONTAINING AN ORCHID EXTRACT, AND COSMETIC CARE METHOD USING SAID COMPOSITION

(30) Priorité: 30.11.2007 FR 0759482
(43) Date de publication de la demande: 11.08.2010
(73) Titulaire: LVMH RECHERCHE, 45800 St. Jean de Braye (FR)
(72) Inventeur: LEPLANQUAIS, Virginie, F-45450 Fay-aux-loges (FR); SAUVAN, Nancy, F-92190 Meudon (FR); PECHER, Virginie, F-45380 La Chapelle Saint Mesmin (FR); GERARD, François, F-73270 Villars-sur-Doron (FR)
(74) Mandataire: Portal, Frédéric
(86) Numéro de dépôt international: PCT/FR2008/052154
(87) Numéro de publication internationale: WO 2009/071855

(56) Documents cités:
- DATABASE CA [Online] CHEMICAL ABSTRACTS SERVICE, COLUMBUS, OHIO, US; SARMA, C. M. ET AL: "Medicinally important orchids of North East India" XP002488801 extrait de STN Database accession no. 2007:1480646 & INDIAN JOURNAL OF ENVIRONMENT AND ECOPLANNING , 13(1-2), 91-100 CODEN: IJEECH; ISSN: 0972-1215, 2007,
- PRASAD D N ET AL: "A study of anti-arthritic action of Vanda roxburghii in albino rats" JOURNAL OF THE INDIAN MEDICAL ASSOCIATION 1966, vol. 46, no. 5, 1966, pages 234-237, XP009102745 ISSN: 0019-5847
- CHAWLA A S ET AL: "Chemical studies and antiinflammatory activity of Vanda roxburghii roots" INDIAN JOURNAL OF PHARMACEUTICAL SCIENCES, vol. 54, no. 4, 1992, pages 159-161, XP009102741 ISSN: 0250-474X cité dans la demande
- NAYAK B S ET AL: "Evaluation of wound healing activity of Vanda roxburghii R.Br (Orchidacea): A preclinical study in a rat model" INTERNATIONAL JOURNAL OF LOWER EXTREMITY WOUNDS 200512 US, vol. 4, no. 4, décembre 2005 (2005-12), pages 200-204, XP009102743 ISSN: 1534-7346
- HUSEN AZAMAL ET AL: "Orchids an important group of plants for traditional system of medicine in India." INDIAN FORESTER, vol. 129, no. 5, mai 2003 (2003-05), pages 651-653, XP009102800 ISSN: 0019-4816
- REDDY K N ET AL: "Ethnobotany of certain orchids of eastern Ghats of Andhra Pradesh" INDIAN FORESTER, vol. 131, no. 1, janvier 2005 (2005-01), pages 90-96, XP009102801 ISSN: 0019-4816
- BULPITT C J: "The uses and misuses of orchids in medicine" QJM, vol. 98, no. 9, septembre 2005 (2005-09), pages 625-631, XP002488800 ISSN: 1460-2725 cité dans la demande

## Description

L'invention concerne une composition cosmétique ou dermatologique contenant un extrait d'orchidée et des méthodes de soin cosmétique utilisant cette composition.

Les orchidées (Orchidaceae) font l'objet de nombreuses recherches dans le domaine du médicament ou de la cosmétique, visant à identifier de nouveaux composés présentant des propriétés intéressantes.

Les orchidées du genre Vanda sont des orchidées épiphytes ou épilithes des forêts tropicales d'Asie et d'Australie.

Ce sont des orchidées monopodiales des forêts de basse altitude à humidité atmosphérique élevée.

Le genre comprend une cinquantaine d'espèces avec beaucoup d'hybrides.

### ETAT DE LA TECHNIQUE

Les vertus thérapeutiques des orchidées du genre Vanda ont été abondamment documentées.

Bulpitt («The uses and misuses of orchids in medicine », QJM, 2005, 98 (9), 625-631) a par exemple rapporté que l'orchidée Vanda coerulea fait partie de la Pharmacopée Indienne, sans donner plus de précisions à ce sujet.

Manandhar et al. (Fitoterapia, 1994, 65 (1), 7-13) ont rapporté l'usage de Vanda cristata dans le district Kaski au Népal, pour soigner les coupures et les blessures par application d'une pâte à base de la plante. Cette orchidée aurait également été utilisée comme expectorant (Husen et al., Orchids an important group of plants for traditional system of medicine in India, Indian Forester, vol.129, n°5, 2003, pages 651-653).

Sigh et al. ont étudié le propriétés médicinales de plantes utilisées par la tribu Tharu du district Nainital dans la région d'Uttar Pradesh en Inde (Int. J. Pharmacogn., 1994 32 1, 51-58). L'application d'une pâte de Vanda tessallata (autre nom de Vanda roxburghii) obtenue à partir de la plante entière, et appliquée avec du sel, soigne les fractures osseuses. L'administration orale d'un extrait aqueux de cette même plante réduirait également les fractures.

Husen et al. (dans Indian Forester, vol.129, n°5, 2003, pages 651-653) ont confirmé l'intérêt de l'orchidée Vanda roxburghii pour le traitement des fractures, mais aussi pour soigner les otites, l'asthme, la syphilis et les douleurs dentaires. Les racines de Vanda tessellata, appliquées localement sous la forme d'une pâte calment les rhumatismes (Reddy et al., Indian Forester, vol.131, n°1, 2005, pages 90-96).

Il a également été décrit dans la demande de brevet japonais JP 2006-257056 qu'un extrait de Vanda roxburghii peut être utilisé comme agent oestrogène pour le traitement du vieillissement de la peau. Par ailleurs, une amélioration de la cicatrisation cutanée a été observée par application d'un extrait aqueux de feuilles de cette même orchidée (Nayak et al., International Journal of Lower Extremity Wounds, vol. 4, n° 4, 2005, pages 200-204).

Chawla et al. (Ind. J. Pharm. Sci., 1992, 54 (4), 159-61) et Prasad et al. (Journal of the Indian Médical Association vol. 46, n°5, 1966, pages 234-237) ont décrit les propriétés anti-inflammatoires de plusieurs extraits de racines de Vanda roxburghii dans des solvants organiques tels que l'éther, le chloroforme ou le méthanol. Les premiers auteurs ont mis en évidence la présence d'un alkylférulate, plus précisément l'acétyltétracosylférulate, dans l'extrait à l'éther, et de béta-silostérol-D-glucoside dans l'extrait au chloroforme. Ces extraits présentent une activité anti-inflammatoire d'intensité variable et inférieure à celle de l'ibuprofène.

L'orchidée Vanda teres a fait l'objet de très peu de publications scientifiques. On notera toutefois que Sarma a rapporté dans Indian Journal of Environment and Ecoplanning (2007), 13 (1-2), 91-100, que des extraits de la plante Papilionanthe teres (synonyne de Vanda teres) ont été utilisés pour traiter la débilité, en les appliquant sur le front des patients pendant les fortes fièvres et que, par ailleurs, des extraits de tige de cette même plante ont pu être utilisés pour protéger du rhume et de la toux.

Les propriétés thérapeutiques d'une autre orchidée, Vanda testacea, ont également été rapportées. Ses feuilles broyées, appliquées en cataplasme, ont été utilisées pour soigner les fractures du bétail (Reddy et al., Indian Forester, vol.131, n°1, 2005, pages 90-96). Husen et al., dans Indian Forester, vol.129, n°5, 2003, pages 651-653, ont mentionné son utilité pour soigner les rhumatismes, les troubles nerveux et les piqûres de scorpions D5.

Il a maintenant été découvert par les inventeurs de la présente invention que, de manière totalement inattendue, les orchidées de l'espèce Vanda teres présentaient de remarquables activités cosmétiques et dermatologiques, en particulier de remarquables propriétés de prévention ou de ralentissement de l'apparition des signes du vieillissement cutané associées à de remarquables propriétés anti-inflammatoires.

La Demanderesse a ainsi montré en particulier qu'un extrait de Vanda teres présentait des propriétés particulièrement intéressantes dans le Vanda teres présentait des propriétés particulièrement intéressantes dans le domaine de la cosmétique, en l'espèce une activité telle qu'elle permet d'utiliser ledit extrait pour contribuer au maintien de l'intégrité de la structure de la peau.

En effet, cet extrait inhibe les enzymes métalloprotéinases MMP-2 et 9 responsables entre autres de la dégradation des fibres de collagène de la matrice extracellulaire, augmente l'expression du gène IL-10.

Il résulte de ces différentes activités, un effet de prévention et de ralentissement de l'apparition des signes du vieillissement de la peau.

Outre ces propriétés inattendues, l'extrait de l'invention présente également une activité anti-inflammatoire par inhibition de la sécrétion de médiateurs de l'inflammation, en particulier l'interleukine 8 (Il-8) et la prostaglandine E2 (PGE₂).

L'extrait obtenu à partir d'orchidées de l'espèce Vanda teres peut ainsi être utilisé en tant qu'agent actif dans une composition cosmétique comprenant au moins un excipient cosmétiquement acceptable, et destinée à être appliquée sur au moins une partie de la peau du visage ou du corps, pour atténuer ou retarder les effets du vieillissement de la peau.

Cet extrait peut être également avantageusement utilisé dans des compositions cosmétiques ou dermatologiques pour ses remarquables propriétés anti-inflammatoires.

### BUTS DE L'INVENTION

La présente invention a pour but principal de fournir un nouvel agent cosmétique ou dermatologique apte à atténuer ou retarder les effets du vieillissement de la peau, en assurant plus particulièrement le maintien de l'intégrité de la structure de la peau, également à lutter contre les inflammations de la peau, et une composition cosmétique ou dermatologique comprenant ledit agent actif.

L'invention a encore pour but de résoudre le problème technique par une solution particulièrement simple, relativement peu coûteuse et utilisable à l'échelle industrielle et cosmétique.

### RESUME DE L'INVENTION

Selon un premier aspect, l'invention concerne une composition cosmétique ou dermatologique contenant au moins un extrait d'orchidée du genre Vanda, obtenu à partir d'au moins une partie d'une orchidée de l'espèce Vanda teres, en solution ou en dispersion dans un véhicule cosmétiquement ou dermatologiquement acceptable compatible avec une application topique sur la peau.

Selon un deuxième aspect, l'invention concerne l'utilisation de cet extrait dans une composition cosmétique ou dermatologique, en tant qu'agent cosmétique ou dermatologique destiné à maintenir la structure de la peau, en particulier en limitant la dégradation de la matrice extracellulaire des couches superficielles du derme et de l'épiderme et/ou à atténuer ou retarder les effets du vieillissement de la peau, en particulier la formation de rides et/ou à obtenir un effet protecteur, correcteur ou restructurant de la peau et/ou en tant qu'agent anti-inflammatoire.

Selon un troisième aspect, l'invention concerne une méthode de soin cosmétique de la peau destinée notamment à obtenir un effet de prévention ou de ralentissement de l'apparition des signes du vieillissement cutané, comprenant l'application sur au moins une partie du corps ou du visage d'une composition cosmétique faisant l'objet du premier aspect de l'invention.

### DESCRIPTION DETAILLEE DE L'INVENTION

La présente invention a ainsi pour premier objet une composition cosmétique ou dermatologique contenant un extrait obtenu à partir d'un matériel végétal formé d'orchidées de l'espèce Vanda teres.

Le matériel végétal utilisé peut être la plante entière ou une partie de la plante telle que les feuilles, la tige, les fleurs ou les racines.

L'extrait est préférentiellement obtenu à partir des tiges et/ou des racines et/ou des feuilles de l'orchidée, de préférence les tiges.

L'extrait est préparé par différents procédés d'extraction connus de l'Homme du Métier.

Toutefois, l'extraction est de préférence réalisée par mise en contact du matériel végétal sélectionné avec un solvant polaire ou un mélange de solvants polaires.

Préalablement à cette étape de mise en contact d'au moins une partie de la plante avec au moins un solvant polaire, cette partie de plante pourra être éventuellement séchée et/ou broyée.

A titre de solvant polaire ou de mélanges de solvants polaires utilisable pour l'étape d'extraction, on choisira avantageusement un solvant ou un mélange de solvants choisi parmi l'eau, un alcool en C1-C4, par exemple l'éthanol, un glycol en C2 à C6 choisi préférentiellement parmi le glycérol, le butylèneglycol et le propylèneglycol et leurs mélanges.

Selon une mise en oeuvre préférée de l'invention, l'extraction est menée en utilisant un mélange hydroalcoolique, en particulier un mélange d'eau et d'éthanol, de préférence un mélange d'eau et d'éthanol respectivement dans un rapport de l'ordre de 90/10 v/v.

Selon une autre variante de l'invention, l'extraction peut également être réalisée par un procédé mettant en oeuvre un solvant polaire à l'état subcritique, ledit solvant étant avantageusement l'eau à l'état subcritique.

Préalablement à l'étape d'extraction elle-même, le matériel végétal peut avoir été séché et/ou broyé.

Selon une mise en oeuvre préférée de l'extraction, le matériel végétal se trouve à l'état sec et broyé.

L'extraction peut aussi comprendre, de façon optionnelle, une étape supplémentaire consistant en un traitement de l'extrait visant à le décolorer partiellement ou complètement, ou à le purifier.

Cette étape de décoloration peut, par exemple, consister en un traitement de l'extrait par une solution d'un solvant ou d'un mélange de solvants polaire, de préférence en un traitement par une solution éthanol/eau dans un ratio de l'ordre de 70/30 v/v, en présence de particules de charbon actif.

L'extraction peut être complétée par une étape d'élimination partielle ou totale des solvants d'extraction.

Dans le premier cas, on concentre généralement l'extrait jusqu'à obtenir un concentré aqueux dépourvu de quantités significatives de solvant organique, dans le second cas on obtient un résidu sec.

De façon alternative, le produit de l'étape d'extraction peut être, lyophilisé ou atomisé pour se présenter sous la forme d'une poudre.

La poudre peut être utilisée en l'état dans une composition cosmétique ou dermatologique selon l'invention ou être redispersée dans un solvant ou un mélange de solvants.

De façon générale, le produit de l'étape d'extraction peut être dissous ou dispersé dans un solvant ou un mélange de solvants, pour être utilisé à titre d'agent actif dans les compositions cosmétiques ou dermatologiques de l'invention.

Le solvant ou le mélange de solvants dans lequel l'extrait est dissous ou dispersé peut être identique ou différent de celui ayant servi à l'extraction.

L'extrait de l'invention peut également être adsorbé sur un support avantageusement choisi parmi les poudres de nylon, poreuses ou non poreuses et les micas ou toute substance minérale lamellaire.

Dans ce cas, l'extrait utilisé est préférentiellement un extrait aqueux.

La composition cosmétique ou dermatologique selon l'invention comprend une quantité efficace d'extrait de l'invention pour obtenir l'effet recherché.

La composition selon l'invention comprend ainsi préférentiellement de 0,001% à 5%, de préférence de 0,01% à 1%, en poids sec d'extrait de Vanda teres.

Les essais réalisés par les inventeurs ont montré que les propriétés des compositions de l'invention liées à la présence de l'extrait de Vanda teres peuvent être également obtenues ou améliorées dans des compositions cosmétiques ou dermatologiques, dans lesquelles l'extrait est associé avec d'autres agents actifs présentant des effets cosmétiques similaires et/ou complémentaires de l'extrait de l'invention.

Ainsi, l'extrait de l'invention, seul ou en association avec d'autres agents actifs participant au maintien de l'intégrité de la structure de la peau, peut être également associé de façon avantageuse avec un ou plusieurs agents actifs choisis parmi les filtres UVA et/ou UVB, les substances antiradicalaires, les substances calmantes, les substances éclaircissantes du teint et /ou régulant les désordres pigmentaires cutanés, les substances présentant un effet hydratant.

Les inventeurs ont également découvert que les remarquables propriétés cosmétiques ou dermatologiques liées à la présence dans la composition d'un extrait d'au moins une partie de la plante de Vanda teres pouvaient être encore, dans bien des cas, grandement améliorées lorsque cette composition contient, en outre, au moins un extrait d'au moins une autre plante appartenant à la famille des orchidées (Orchidaceae).

Cet extrait supplémentaire d'une autre plante appartenant à la famille des orchidées pourra être en particulier un extrait d'au moins une orchidée du genre Vanda et, tout particulièrement un extrait d'au moins une partie d'une orchidée de l'espèce Vanda denisoniana et/ou d'au moins une partie d'une orchidée de l'espèce Vanda coerulea.

Les compositions de l'invention pourront comprendre, à la fois, un extrait d'au moins une partie de la plante Vanda teres associé à au moins un extrait d'au moins une partie d'une autre orchidée du genre Vanda telle que définie ci-dessus et éventuellement au moins un autre extrait d'une autre plante appartenant à la famille des orchidées.

Selon une variante particulièrement avantageuse, les compositions de l'invention comprennent au moins un extrait de tige de Vanda teres et/ou un extrait de racine de Vanda teres, en combinaison avec un extrait de tige de Vanda denisoniana et/ou un extrait de tige de Vanda coerulea.

Les essais effectués par les inventeurs ont en effet montré que la présence dans la même composition d'au moins un extrait de Vanda teres associé à au moins un extrait d'une autre orchidée du genre Vanda conduisait dans de nombreux cas à des propriétés cosmétiques ou dermatologiques améliorées.

Les inventeurs ont pu même, dans certains cas, mettre en évidence de véritables synergies observées par mélange d'au moins un extrait de Vanda teres avec au moins un autre extrait d'au moins une partie d'au moins une autre orchidée du genre Vanda.

Ainsi, les essais réalisés par les inventeurs ont pu permettre de mettre en évidence que les extraits de racines de Vanda teres, même si ces parties ne sont pas en elles-mêmes les plus actives de la plante, amélioraient considérablement les propriétés de prévention ou de ralentissement de l'apparition des signes du vieillissement cutané ou les propriétés anti-inflammatoires observées avec des extraits de plante du genre Vanda.

Comme cela ressort des exemples ci-après, à titre d'exemple d'associations qui améliorent de façon significative voire même qui réalisent une véritable synergie, on citera les associations d'extraits de tiges de Vanda teres avec des extraits de tiges et/ou de racines de Vanda denisoniana et/ou avec des extraits de tiges de Vanda coerulea.

On notera en particulier que de véritables effets de synergie ont pu être observés, aussi bien en ce qui concerne l'activité de prévention ou de ralentissement de l'apparition des signes du vieillissement cutané que l'activité anti-inflammatoire en mélangeant dans la composition, un extrait de tiges de Vanda teres, un extrait de racines de Vanda teres, un extrait de tiges de Vanda denisoniana et un extrait de tiges de Vanda coerulea et cela, dans des proportions qui peuvent varier grandement.

Outre l'extrait de l'invention, ladite composition cosmétique ou dermatologique comprend au moins un excipient cosmétiquement ou dermatologiquement acceptable qui peut être choisi parmi des pigments, des colorants, des polymères, des agents tensioactifs, des agents de rhéologie, des parfums, des électrolytes, des ajusteurs de pH, des agents anti-oxydants, des conservateurs, et leurs mélanges.

La composition cosmétique ou dermatologique selon l'invention peut être par exemple un sérum, une lotion, une émulsion, une crème, en particulier une crème teintée ou bien encore un hydrogel, de préférence un masque, ou se présenter sous la forme d'un stick, ou encore d'un patch.

Les compositions de l'invention présentent un effet particulièrement recherché pour atténuer ou retarder les effets du vieillissement de la peau, et permettent en particulier d'obtenir un effet protecteur, correcteur, restructurant, lorsqu'on les applique sur la peau du visage ou du corps.

La présente invention concerne, comme exposé précédemment, une utilisation des extraits tels que définis ci-dessus en tant qu'agent cosmétique ou dermatologique destiné à maintenir la structure de la peau, en particulier en limitant la dégradation de la matrice extracellulaire des couches superficielles du derme et de l'épiderme et/ou à atténuer ou retarder les effets du vieillissement de la peau, en particulier la formation de rides et/ou à obtenir un effet protecteur, correcteur ou restructurant de la peau et/ou en tant qu'agent anti-inflammatoire.

Comme cela ressort clairement des exemples ci-après, la demanderesse a mis en évidence l'intérêt des extraits de la plante Vanda teres associés éventuellement, en outre, à d'autres extraits de Vanda pour améliorer les activités listées ci-dessous :
- activité anti-inflammatoire par inhibition de la sécrétion de médiateurs de l'inflammation IL-8 et PGE₂,
- propriétés anti-radicalaires, préservant la peau des dommages causés par les stress oxydatifs,
- synthèse des fibres de fibronectine et de collagène de type I, fibres de la matrice extracellulaire permettant le maintien de la structure de la peau,
- inhibition des enzymes MMP-2 et 9 dégradant les fibres de la matrice extracellulaire

Ainsi, l'invention porte également sur une utilisation des extraits de Vanda teres définis précédemment comme agent cosmétique ou dermatologique ou pour la fabrication d'une composition cosmétique destinée à maintenir la structure de la peau, en particulier en limitant la dégradation de la matrice protéique extracellulaire des couches superficielles du derme et de l'épiderme, et ainsi atténuer ou retarder les effets du vieillissement de la peau, en particulier la formation de rides, ou encore à obtenir un effet protecteur, correcteur, restructurant.

L'invention a également pour objet une méthode de soin cosmétique utilisant les compositions cosmétiques comprenant une quantité efficace dudit extrait pour contribuer au maintien de l'intégrité de la structure de la peau, en particulier par stimulation de la synthèse de la fibronectine, par inhibition des enzymes MMP-2 et 9 responsables de la dégradation des fibres de collagène et par augmentation de l'expression du gène codant pour linterleukine IL-10.

Ladite méthode de soin cosmétique comprend l'application sur au moins une zone concernée de la peau du visage ou du corps, d'une composition telle que décrite précédemment et comprenant un extrait tel que précédemment défini.

Dans les exemples, tous les pourcentages sont donnés en poids, la température est en degrés Celsius, la pression est la pression atmosphérique, sauf indication contraire.

Outre des orchidées du genre Vanda, le matériel végétal à partir duquel est préparé l'extrait de l'invention peut comprendre un ou plusieurs extraits d'autres plantes, sous forme d'extraits de plantes entières ou de parties de plantes.

Ces plantes peuvent être soit des orchidées d'un genre différent du genre Vanda, soit des plantes d'une autre famille, connues pour avoir des propriétés similaires ou complémentaires à celles mises en évidence pour les orchidées du genre Vanda.

On pourra choisir en particulier des plantes dont les extraits sont connus pour ralentir ou prévenir les effets du vieillissement de la peau, par divers mécanismes tels que le maintien de de la structure de la peau ou une action sur les rides.

D'autres buts, caractéristiques et avantages de l'invention apparaîtront clairement à la lumière de la description explicative qui va suivre faite en référence aux exemples de préparation d'extraits, et à des exemples de compositions cosmétiques utilisant de tels extraits, donnés simplement à titre d'illustration.

### EXEMPLES

### I. PREPARATION D'EXTRAITS

### Exemple 1 : préparation d'un extrait de tiges de Vanda teres (extrait n° 1 selon l'invention)

Le matériel végétal (tiges de la plante) à l'état sec est broyé extemporanément.

On introduit 10g de végétal broyé dans un ballon de 250 mL dans lequel on ajoute 150mL d'un mélange éthanol/eau (90/10 v/v).

On met le ballon surmonté d'un réfrigérant à boule sous agitation magnétique dans un bain thermostaté, puis on chauffe jusqu'au reflux de solvant.

Le reflux est maintenu 30 min sous agitation.

Une fois le chauffage arrêté, on laisse refroidir le ballon à température ambiante hors du bain.

Le mélange est ensuite filtré sous vide sur buchner avec un filtre GF/F Whatman 70µm et une fiole tarée : on obtient le filtrat 1.

Le gâteau est lavé sur le buchner avec 50 mL du solvant d'extraction : on obtient le filtrat 2.

Les 2 filtrats sont réunis, pesés.

Le filtrat ainsi obtenu est introduit dans un ballon préalablement taré, puis concentré à sec à l'évaporateur rotatif dans un bain d'eau placé à une température maximale de 50°C.

On obtient un résidu sec qui est quantifié pour déterminer le rendement massique d'extraction exprimé par rapport à la masse de végétal sec introduit.

Le rendement d'extraction exprimé en masse d'extrait sec obtenu pour 100 g de matière végétale à l'état broyé sec de départ est de 12,4.

### Exemple 2 : Préparation d'un extrait de racines de Vanda teres (extrait n° 2 selon l'invention)

On utilise le même protocole que celui utilisé pour la préparation de l'extrait n° 1 de l'exemple 1 mais on l'applique à un matériel végétal constitué de racines de Vanda teres.

On obtient un extrait ci-après désigné par extrait n° 2 de l'invention, avec un rendement d'extraction de 14,8.

### Exemple 3 : Préparation d'autres extraits de parties de plante du genre Vanda

On utilise le même protocole d'extraction que celui défini dans l'exemple 1 pour préparer différents extraits de Vanda teres et Vanda coerulea.
- extrait de tige de Vanda denisoniana, (rendement d'extraction de 10,6)
- extrait de tige de Vanda coerulea, (rendement d'extraction de 8,9)
- extrait de racines de Vanda coerulea

Ces différents extraits seront utilisés dans les exemples qui suivent soit séparément, soit en mélange et combinés dans différentes proportions avec l'extrait de l'exemple 1 et/ou avec l'extrait de l'exemple 2.

### II. PROTOCOLE, MATERIEL ET METHODE DES TESTS D'ACTIVITE

Les doses d'utilisation des extraits sont au préalable déterminées par le test XTT (réactif : sel de Tétrazolium) sur culture cellulaire de deux lignées de cellules, des cellules HaCaT (lignée de kératinocytes immortalisés in vitro) et de fibroblastes humains normaux (FHN).

### 1. Marqueurs de l'inflammation

### 1-1) dosage de /7nterleukine-8 (IL-8)

L'inflammation est une réponse physiologique normale et immédiate à toute agression de nature mécanique ou infectieuse.

Une production excessive en radicaux libres de l'oxygène (ROS) entraîne la libération de certaines cytokines responsables du phénomène pro-inflammatoire, aboutissant à une accélération du vieillissement cutané.

Une augmentation de l'interleukine-8 (IL-8) peut alors être observée au niveau de l'épiderme : les kératinocytes participent activement à la défense immunitaire en sécrétant des cytokines, dont l'IL-8 qui crée un gradient chimiotactique attirant les cellules impliquées dans l'inflammation : lymphocytes T, neutrophiles... Il a été démontré que les kératinocytes expriment et libèrent l'IL-8, sous stimulation avec l'interieukine-1β.

### • protocole

Les cellules HaCaT sont ensemencées à raison de 10000 cellules/puits (milieu de culture KSFM complémenté, Gibco) dans une microplaque à 96 puits. La plaque est placée à l'étuve (37°C et 5% CO2) pour 24 heures.

Les extraits testés, aux doses d'utilisation préalablement déterminées par le test XTT, sont mis en présence des cellules après dilution adéquate dans le milieu de culture.

En parallèle, les cellules sont stimulées avec l'IL-1β à 25 ng/mL (R&D Systems).

Une colonne est traitée avec le cholécalciférol à 0,39 µg/mL (Sigma) et stimulée (afin d'obtenir le témoin positif), et une colonne est non traitée et stimulée avec l'IL-1β.

La colonne de cellules non traitée est mise en présence du solvant de solubilisation DMSO (condition témoin).

Chaque concentration de produit, le témoin positif et le témoin sans traitement, sont évalués sur 4 puits de kératinocytes humains normaux (KHN).

Après 24 heures de traitement, les surnageants de culture sont prélevés et stockés à -20°C.

Le dosage s'effectue selon le protocole décrit dans le kit « Human IL-8/ NAP-1 ELISA » (Abcys).

Ce test permet de doser, par mesure spectrophotométrique à 450 nm, la quantité d'IL-8 présente dans les surnageants, proportionnelle à l'absorbance mesurée.

### 1-2) dosage des prostaglandines E2 (PGE2)

L'augmentation du taux de PGE2 peut se faire sous certaines conditions pathologiques, comme l'inflammation ou certains cancers.

PGE2 est le médiateur majeur des phénomènes de réponses inflammatoires lors de dommages tissulaires.

### • protocole

Les cellules HaCaT sont ensemencées à raison de 10000 cellules/puits (milieu de culture KSFM complémenté, Gibco) dans une microplaque à 96 puits.

La plaque est placée à l'étuve (37°C et 5% CO2) pour 24 heures.

Les extraits testés sont mis en présence des cellules, après dilution adéquate dans le milieu de culture.

Une colonne est traitée avec l'indométhacine à 1 µg/mL (Sigma) afin d'obtenir le témoin positif, et une colonne est non traitée mais mise en présence du DMSO.

Chaque concentration de produit, le témoin positif et le témoin sans traitement, sont évalués sur 4 puits de KHN.

Après 24 heures de traitement, les surnageants de culture sont prélevés et stockés à -20°C.

Le dosage s'effectue selon le protocole décrit dans le kit de dosage PGE2 de R&D Systems.

Cette technique permet de doser, par mesure spectrophotométrique à 450 nm, la quantité de PGE2 présente dans les surnageants qui est inversement proportionnelle à l'absorbance mesurée.

### 2. Mesure de l'expression du messager de l'interleukine-10 (IL-10)

L'Il-10, décrite comme un facteur inhibant la production de cytokines par des lymphocytes Th1 et les fonctions effectrices des monocytes/macrophages, est en fait une cytokine capable d'agir sur de nombreuses cibles.

Sa fonction principale semble être l'inhibition de la réponse inflammatoire.

L'Il-10 régule également la prolifération et la différenciation de certaines cellules de l'inflammation ou encore des kératinocytes.

### • protocole

L'effet des extraits d'orchidées est évalué par la méthode RT-QPCR sur kératinocytes humains en monocouche (NHEK) à confluence normale (confluence standard biologie moléculaire), en milieu SFM à faible taux de calcium sans compléments, en formats adaptés.

Les extraits et le milieu contrôle sont testés pendant 24 heures, puis l'extraction d'ARN total est réalisée, suivie par des traitements DNAse et une réverse-transcription selon les protocoles standards.

Une PCR quantitative en triplicate a permis de mesurer l'expression du marqueur IL-10 rapportée au marqueur de référence G3PDH.

### 3. Marqueurs du maintien de la structure de la peau

Les tests sont faits sur fibroblastes humains normaux (FHN), cellules du derme favorisant le maintien de la structure de la peau.

Les FHN sont ensemencés à la densité de 5000 cellules/puits et 200 µL/puits de milieu de culture MEM (Gibco Invitrogen) complété avec de la glutamine (Gibco Invitrogen, concentration finale 2mM) et 10% de SVF (sérum de veau foetal), dans une microplaque 96 puits (Falcon).

Les bords de la plaque ne contiendront pas de cellules, et la plaque de culture est placée à l'étuve pendant 24 heures afin d'obtenir 80% de confluence cellulaire.

### 3-1) dosage des enzymes MMP-2 et 9

Les enzymes "Matrix metalloproteinases" (MMPs) ont pour fonction la dégradation des protéines de la matrice extracellulaire.

Elles jouent un rôle important dans de nombreux processus physiologiques normaux comme le développement embryonnaire, la morphogenèse, et le remodelage tissulaire.

MMP-2, encore appelée gélatinase A, est exprimée par les cellules du mésenchyme (principalement les fibroblastes) pendant le développement et la régénération tissulaire. Cette expression est étroitement associée aux cellules type neutrophiles, macrophages et monocytes.

Avec MMP-9, cette protéine dégrade le collagène type IV, composé majeur des membranes basales et de la gélatine (collagène dénaturé).

Elle peut aussi dégrader d'autres types de collagènes (V, VII et X), l'élastine et la fibronectine.

MMP-9, appelée gélatinase B, possède 3 domaines fibronectine type II et un domaine homologue au collagène type V.

Pro-MMP-9 peut être activée par MMP-3 ou par certaines protéinases bactériennes.

Les substrats de MMP-9 peuvent être des collagènes natifs type IV, V, VII, X et XI, la fibronectine...

### • protocole

Les extraits, aux doses d'utilisation préalablement déterminées par le test XTT, sont mis en présence des cellules après dilution adéquate dans du milieu de culture.

Une colonne est traitée avec l'anogelline à 25 µg/mL, et une colonne est mise en présence du solvant de solubilisation DMSO (condition témoin).

Chaque concentration de produit, le témoin positif et le témoin sans traitement, sont évalués sur 4 puits de KHN.

Après 48 heures de traitement, les surnageants de culture sont prélevés et stockés à -20°C.

Le dosage de MMP-2 et 9 s'effectue selon les protocoles décrits dans les kits Quantikine de R&D Systems qui permettent de doser respectivement, par mesure spectrophotométrique à 450 nm, les quantités de MMP-2 et MMP-9 présentes dans les surnageants.

### 3-2) dosage des fibres de collagène I et fibronectine

### • protocole

Comme précédemment, les extraits sont mis en présence des cellules après dilution adéquate dans du milieu de culture.

Une colonne est traitée avec de la vitamine C (Acide ascorbique, Sigma) 0.1 µM, et une colonne est mise en présence du solvant de solubilisation DMSO (condition témoin).

Chaque concentration de produit, le témoin positif et le témoin sans traitement, sont évalués sur 4 puits de KHN.

Après 48 heures de traitement, les surnageants de culture sont prélevés et stockés à -20°C.

Le dosage s'effectue selon les protocoles décrits dans les kits EIA de Takara.

Ces 2 tests utilisent une technique immunoenzymatique permettant de doser respectivement, par mesure spectrophotométrique à 450 nm, les quantités de fibres de collagène et fibronectine présentes dans les surnageants.

### 4. Propriétés anti-radicalaires

On réalise sur les extraits, le test TBARs qui mesure le taux de protection des lipides membranaires des cellules en culture.

Les HaCaT sont ensemencées à 67 passages dans des plaques 24 puits à raison de 75 000 cellules et 1mL par puits. Après 24 heures d'incubation à l'étuve (37°C, 5% CO2) correspondant à 80% de confluence, les cellules sont mises en présence des extraits d'orchidées.

On prépare également un témoin constitué par des plaques de cellules non traitées par des extraits d'orchidées.

Le traitement se fait en triplicate et à 1% (1µL/mL d'échantillon), pour une durée de 24 heures (37°C, 5% CO2).

Deux plaques sont travaillées de la même manière, à la fois pour le traitement par l'extrait et pour le témoin, afin d'irradier l'une des deux plaques aux UVA à raison de 12 J/cm² (changement du milieu de culture par du PBS pour les deux plaques).

A la fin de l'irradiation, les surnageants des deux plaques sont éliminés, 500 µL de SDS 0,8% est ajouté sur chaque puits de cellules.

Après 5 minutes de contact, la récupération des cellules se fait par grattage des puits.

250 µL de cellules sont mis en présence de 375 µL d'acide thiobarbiturique 0,8% et 375 µL d'acide acétique 20% dans des tubes pyrex à bouchon, portés au bain-marie à 95°C pendant une heure.

Après une dernière étape de centrifugation, le surnageant est récupéré afin de lire son absorbance à 532 nm.

On mesure l'absorbance pour l'échantillon soumis au rayonnement UV, ainsi que pour l'échantillon non irradié qui est corrigée de la façon suivante :
- Absorbance corrigée = (Absorbance mesurée - Absorbance de l'eau)

Pour le pourcentage d'inhibition de la lipoperoxydation, le calcul se fait de la façon suivante :
- Absorbance corrigée (témoin) = (Absorbance corrigée (solvant UV) - Absorbance corrigée (solvant))

Le pourcentage d'inhibition pour chaque échantillon s'obtient grâce à la formule : (Absorbance corrigée (UV échantillon) - Absorbance corrigée (échantillon non traité)) / Absorbance corrigée (témoin).

### III. ACTIVITES DES EXTRAITS DE L'INVENTION

### 1. Activité anti-inflammatoire

### Inflammation (HaCat) - dosage d'IL-8 et de PGE₂

Les extraits sont testés à des doses d'utilisation préalablement déterminées par le test XTT qui sont respectivement de :
- 25 µg/mL pour les racines de la plante,
- 50 µg pour les tiges de la plante,
- 12,5 µg pour les feuilles.

Les pourcentages d'inhibition de libération d'IL-8 et PGE₂ sont calculés pour chaque traitement avec un extrait d'orchidées, par rapport à la quantité basale d'IL-8 présente dans les surnageants de cultures non traitées.

Les résultats sont mentionnés dans le tableau ci-dessous :

| genre | espèce | organe | IL8 | PGE2 |
|---|---|---|---|---|
| Vanda | teres | tiges | -30% | -33% |
| Vanda | teres | feuilles | -14% | -67% |
| Vanda | teres | racines | -8% | -57% |

On constate une diminution significative de la libération des marqueurs d'IL-8 et PGE₂.

Cet effet d'inhibition sur la libération de ces marqueurs de l'inflammation traduit une activité anti-inflammatoire de l'extrait testé.

Cette activité anti-inflammatoire bloque le phénomène pro-inflammatoire provoqué par l'excès de production de radicaux libres et évite ainsi l'accélération du vieillissement cutané.

### 2. Tests anti-radicalaires

Le test réalisé sur un extrait de tiges de Vanda teres indique une inhibition de la peroxydation de 41%.

### 3. Dosage du pro-collagène de type I, de la fibronectine et activité MMP-2 et 9 (FHH)

Les extraits sont testés à la dose de 25 µg/mL.

Les résultats sont regroupés dans le tableau ci-dessous :

| genre | espèce | organe | collagène | fibronectine | MMP2 | MMP9 |
|---|---|---|---|---|---|---|
| Vanda | teres | tiges | 8% | 28% | -45% | -36% |
| Vanda | teres | feuilles | 27% | 0 | -34% | -15% |
| Vanda | teres | racines | -20% | 0 | -43% | -10% |

Le traitement des cellules par chacun des extraits testés se traduit par une diminution importante de la synthèse des enzymes métalloprotéinases responsable de la dégradation de la matrice extracellulaire formée en particulier par le collagène.

Les extraits présentent une activité d'inhibition vis-à-vis des métalloprotéinases, se traduisant par un effet cosmétique de prévention du vieillissement cutané par un ralentissement de la dégradation de la matrice extracellulaire.

On notera en outre, que l'extrait de tiges a montré une action significative sur la synthèse du collagène et de la fibronectine et que les extraits de feuilles ont montré une activité particulièrement significative pour la synthèse du collagène.

### 4. Expression du gène codant pour l'IL-10 (KHN)

Un extrait de tiges de Vanda teres conduit à une augmentation significative de 1000% de l'expression de ce gène par rapport à une expression du témoin non traité.

### Conclusions des essais

Il ressort des essais ci-dessus que les extraits de tiges présentent un excellent potentiel en terme d'activité biologique globale de prévention ou de ralentissement de l'apparition des signes du vieillissement cutané, compte tenu des résultats obtenus sur les différents marqueurs.

### IV. ACTIVITE BIOLOGIQUE DE MELANGES D'EXTRAITS DU GENRE VANDA

On a réalisé dans cet exemple une étude systématique de l'activité de prévention ou de ralentissement de l'apparition des signes du vieillissement cutané et de l'activité anti-inflammatoire d'un grand nombre de mélanges des quatre types d'extraits suivants :
- extrait de racines de Vanda teres,
- extrait de tiges de Vanda denisoniana,
- extrait de tiges de Vanda coerulea,
- extrait de tiges de Vanda teres.

Les différents essais ont été réalisés avec une concentration totale en extraits de 25 µg/mL pour chacun des tests, de façon à pouvoir comparer l'efficacité des différents mélanges aux efficacités obtenues avec chacun des types d'extraits pris seuls.

Pour réaliser cette étude, deux modèles biologiques ont été plus particulièrement retenus :
- expression de MMP-2 dans des surnageants de culture de FHN,
- expression de PGE2 dans des surnageants de culture de HaCAT.

Chaque mélange ou extrait testé est préparé de façon à obtenir une concentration correspondant à 25 µg/mL.

Pour chaque expérience, les valeurs du témoin positif et les valeurs des extraits sont comparées en utilisant le test de Student. Leur différence est dite significative quand la valeur est inférieure ou égale à 0.05.

Les essais réalisés ont permis de caractériser un certain nombre de mélanges particulièrement intéressants présentant des activités significatives vis-à-vis de chacun des deux modèles étudiés, voire même des véritables effets de synergie.

A titre de mélanges intéressants, on citera un mélange contenant un extrait de racines de Vanda teres, un extrait de tiges de Vanda teres et un extrait de tiges de Vanda denisoniana dans des proportions équivalentes (1/3 de chaque extrait), ainsi qu'un mélange d'un extrait de racines de Vanda teres et d'un extrait de tiges de Vanda denisoniana dans des proportions équivalentes (50/50).

On citera également un mélange contenant les quatre extraits cités ci-dessus dans des proportions équivalentes (25%) ainsi qu'un mélange présentant la composition suivante :
- racines de Vanda teres : 12,5%,
- tiges de Vanda denisoniana : 62,5%,
- tiges de Vanda coerulea : 12,5%,
- tiges de Vanda teres : 12,5%.

Tous ces mélanges s'avèrent particulièrement intéressants sur chacun des deux modèles.

### V. COMPOSITIONS COSMETIQUES DE L'INVENTION

### Exemple 4 - Composition cosmétique comprenant l'extrait de l'invention

L'extrait d'orchidées utilisé à titre d'agent actif dans la composition cosmétique de cet exemple, est obtenu en reproduisant le procédé de l'exemple 1 mais en l'appliquant à un mélange de tiges de Vanda denisoniana, de Vanda teres et de Vanda coerulea, à l'état sec et broyé et suivant un ratio 1/1/1 de ces trois plantes.

L'extrait sec ainsi obtenu est mis ensuite en solution à 1% p/p dans un mélange glycérol/eau 60/40 v/v.

Cette solution est utilisée à titre d'agent actif pour la préparation de la composition cosmétique suivante (% exprimé en p/p) :

**Phase A**

| | |
|---|---|
| Solution à 1% en extrait d'orchidées | 0,3% |
| Phénoxyéthanol | 0,5% |
| Gomme xanthane | 0,2% |
| Acrylates/C20-30 alkylacrylate crospolymères | 0,15% |
| EDTA tétrasodique | 0,1% |
| Eau | qs |

**Phase B**

| | |
|---|---|
| Polyisobutène hydrogéné | 4% |
| Squalane | 3% |
| Caprylique/caprique triglycéride | 3%% |
| Pentylène glycol | 3% |
| Glycéryl stéarate | 3% |
| PEG-100 stéarate | 2,5% |
| Cire d'abeilles | 1,5% |
| Dicaprylyl carbonate | 1,5% |
| Cétyl alcool | 1% |
| Stéaryl alcool | 1% |
| Diméthicone | 1% |

**Phase C**

| | |
|---|---|
| Hydroxyde de sodium | 0,04% |
| Eau | qs 100% |

On disperse les gélifiants de la phase A dans l'eau puis on chauffe à 80-85°C, avant de solubiliser tous les autres composés y compris la solution d'extrait d'orchidées.

Les composés de la phase B sont chauffés à 85°C pour former une phase homogène.

On émulsionne la phase A dans la phase B à l'aide d'un mélangeur Ystral.

L'émulsion huile/eau ainsi obtenue est finalement neutralisée à l'aide d'une solution aqueuse d'hydroxyde de sodium % p/p, puis refroidie.

La composition obtenue est une crème destinée à être appliquée sur le visage ou une partie du visage.

## Revendications

1. Composition cosmétique sous la forme d'un sérum, d'une lotion, d'une émulsion, d'une crème, d'une crème teintée, d'un hydrogel, d'un masque, d'un stick ou d'un film patch, **caractérisée en ce qu'**elle contient
- de 0,001 % à 5 % en poids sec d'au moins un extrait d'au moins une partie d'une orchidée de l'espèce Vanda teres, en solution ou en dispersion dans un véhicule cosmétiquement acceptable compatible avec une application topique sur la peau, et
- au moins un excipient choisi parmi des pigments, des colorants, des polymères, des agents tensioactifs, des agents de rhéologie, des parfums, des ajusteurs de pH, des agents anti-oxydants et des conservateurs.

2. Composition selon la revendication 1, **caractérisée en ce que** ledit extrait est obtenu à partir de la tige et/ou des racines et/ou des feuilles de l'orchidée Vanda teres.

3. Composition selon l'une des revendications 1 ou 2, **caractérisée en ce que** ledit extrait est un extrait de tige.

4. Composition selon l'une des revendications 1 à 3, **caractérisée en ce que** ledit extrait est obtenu par mise en contact d'au moins une partie de ladite orchidée, éventuellement séchée et/ou broyée avec au moins un solvant polaire.

5. Composition selon la revendication 4 **caractérisée en ce que** ledit solvant polaire est choisi dans le groupe constitué de l'eau, des alcools en C1-C4, en particulier de l'éthanol, des glycols en C2 à C6, en particulier le glycérol, le butylèneglycol et le propylèneglycol.

6. Composition selon la revendication 5, **caractérisée en ce que** ledit solvant est un mélange hydro-éthanolique, de préférence un mélange comprenant de l'ordre de 90% d'eau pour 10% d'éthanol, en volume.

7. Composition selon la revendication 4, **caractérisée en ce que** ledit solvant polaire est un solvant à l'état subcritique, en particulier l'eau à l'état subcritique.

8. Composition selon l'une des revendications 1 à 7, **caractérisée en ce que** ledit extrait a été soumis à au moins une étape de décoloration et/ou de purification.

9. Composition selon l'une des revendications 1 à 8, **caractérisée en ce que** ledit extrait est introduit dans ladite composition après avoir été absorbé sur un support, notamment sur un support comprenant des poudres de nylon, poreuses ou non poreuses, ou des micas ou une substance minérale lamellaire.

10. Composition selon l'une des revendications 1 à 9, **caractérisée en ce que** ledit extrait est un extrait aqueux.

11. Composition selon l'une des revendications 1 à 10, **caractérisée en ce qu'**elle contient de 0,01 à 1% en poids sec dudit extrait tel que défini dans l'une des revendications 1 à 8.

12. Composition selon l'une des revendications 1 à 11, **caractérisée en ce qu'**elle contient, en outre, au moins un extrait d'au moins une autre plante appartenant à la famille des orchidées (Orchidaceae).

13. Composition selon l'une des revendications 1 à 12, **caractérisée en ce qu'**elle contient, en outre, au moins un extrait d'au moins une autre orchidée du genre Vanda, en particulier un extrait d'au moins une partie d'une orchidée de l'espèce Vanda denisoniana et/ou d'au moins une partie d'une orchidée de l'espèce Vanda coerulea.

14. Composition selon la revendication 13, **caractérisée en ce qu'**elle contient au moins un extrait de tige de Vanda teres et/ou un extrait de racines de Vanda teres, en combinaison avec un extrait de tige de Vanda denisoniana et/ou un extrait de tige de Vanda coerulea.

15. Composition selon l'une des revendications 1 à 14, **caractérisée en ce qu'**elle comprend un extrait de racine de Vanda teres.

16. Utilisation, dans une composition cosmétique, d'un extrait tel que défini dans l'une des revendications 1 à 8 en tant qu'agent cosmétique destiné à maintenir la structure de la peau, en particulier en limitant la dégradation de la matrice extracellulaire des couches superficielles du derme et de l'épiderme et/ou à atténuer ou retarder les effets du vieillissement de la peau, en particulier la formation de rides et/ou à obtenir un effet protecteur, correcteur ou restructurant de la peau.

17. Méthode de soin cosmétique de la peau destinée notamment à obtenir un effet de prévention ou de ralentissement de l'apparition des signes du vieillissement cutané, **caractérisée en ce qu'**elle comprend l'application sur au moins une partie du corps ou du visage d'une composition cosmétique telle que définie dans l'une des revendications 1 à 15.

## Patentansprüche

1. Kosmetische Zusammensetzung in der Form eines Serums, einer Lotion, einer Emulsion, einer Creme, einer gefärbten Creme, eines Hydrogels, einer Maske, eines Sticks oder eines Pflasterfilms, **dadurch gekennzeichnet, dass** diese enthält:
- von 0,001 % bis 5 %, bezogen auf das Trockengewicht, mindestens eines Extrakts mindestens eines Teils einer Orchidee der Art Vanda teres, in Lösung oder in Dispersion in einem kosmetisch annehmbaren Träger, der mit einer topischen Anwendung auf der Haut kompatibel ist, und
- mindestens einen Exzipienten, ausgewählt aus Pigmenten, Farbstoffen, Polymeren, grenzflächenaktiven Stoffen, Rheologiemitteln, Parfums, pH-Einstellmitteln, Antioxidantien und Konservierungsmitteln.

2. Zusammensetzung nach Anspruch 1, **dadurch gekennzeichnet, dass** der Extrakt aus dem Stiel und/oder den Wurzeln und/oder den Blättern der Orchidee Vanda teres erhalten wird.

3. Zusammensetzung nach einem der Ansprüche 1 oder 2, **dadurch gekennzeichnet, dass** der Extrakt ein Stielextrakt ist.

4. Zusammensetzung nach einem der Ansprüche 1 bis 3, **dadurch gekennzeichnet, dass** der Extrakt durch Inkontaktbringen mindestens eines Teils der Orchidee, gegebenenfalls getrocknet und/oder zerkleinert, mit mindestens einem polaren Lösungsmittel erhalten wird.

5. Zusammensetzung nach Anspruch 4, **dadurch gekennzeichnet, dass** das polare Lösungsmittel ausgewählt ist aus der Gruppe bestehend aus Wasser, C1-C4-Alkoholen, insbesondere Ethanol, C2-C6-Glykolen, insbesondere Glycerin, Butylenglykol und Propylenglykol.

6. Zusammensetzung nach Anspruch 5, **dadurch gekennzeichnet, dass** das Lösungsmittel eine hydro-ethanolische Mischung ist, vorzugsweise eine Mischung, die in der Größenordnung von 90 % Wasser für 10 % Ethanol, bezogen auf das Volumen, umfasst.

7. Zusammensetzung nach Anspruch 4, **dadurch gekennzeichnet, dass** das polare Lösungsmittel ein Lösungsmittel im subkritischen Zustand ist, insbesondere Wasser im subkritischen Zustand.

8. Zusammensetzung nach einem der Ansprüche 1 bis 7, **dadurch gekennzeichnet, dass** der Extrakt mindestens einem Schritt der Entfärbung und/oder der Reinigung unterzogen wurde.

9. Zusammensetzung nach einem der Ansprüche 1 bis 8, **dadurch gekennzeichnet, dass** der Extrakt in die Zusammensetzung eingebracht wird, nachdem er auf einem Träger absorbiert wurde, insbesondere auf einem Träger, der poröse oder nicht poröse Pulver von Nylon, oder Micas oder eine lamellare mineralische Substanz umfasst.

10. Zusammensetzung nach einem der Ansprüche 1 bis 9, **dadurch gekennzeichnet, dass** der Extrakt ein wässriger Extrakt ist.

11. Zusammensetzung nach einem der Ansprüche 1 bis 10, **dadurch gekennzeichnet, dass** diese von 0,01 % bis 1 %, bezogen auf das Trockengewicht, des Extrakts, wie in einem der Ansprüche 1 bis 8 definiert, umfasst.

12. Zusammensetzung nach einem der Ansprüche 1 bis 11, **dadurch gekennzeichnet, dass** diese außerdem mindestens einen Extrakt mindestens einer anderen Pflanze enthält, die zur Familie der Orchideen (Orchidaceae) gehört.

13. Zusammensetzung nach einem der Ansprüche 1 bis 12, **dadurch gekennzeichnet, dass** diese außerdem mindestens einen Extrakt mindestens einer anderen Orchidee der Gattung Vanda enthält, insbesondere einen Extrakt mindestens eines Teils einer Orchidee der Art Vanda denisoniana und/oder mindestens eines Teils einer Orchidee der Art Vanda coerulea.

14. Zusammensetzung nach Anspruch 13, **dadurch gekennzeichnet, dass** diese mindestens einen Stielextrakt von Vanda teres und/oder einen Wurzelextrakt von Vanda teres in Kombination mit einem Stielextrakt von Vanda denisoniana und/oder einem Stielextrakt von Vanda coerulea enthält.

15. Zusammensetzung nach einem der Ansprüche 1 bis 14, **dadurch gekennzeichnet, dass** diese einen Wurzelextrakt von Vanda teres umfasst.

16. Verwendung, in einer kosmetischen Zusammensetzung, eines Extrakts, wie in einem der Ansprüche 1 bis 8 definiert, als kosmetisches Mittel, das bestimmt ist zur Aufrechterhaltung der Struktur der Haut, insbesondere durch Einschränkung des Abbaus der extrazellulären Matrix der Oberflächenschichten der Dermis und der Epidermis, und/oder zur Abschwächung oder Verlangsamung der Effekte der Alterung der Haut, insbesondere der Bildung von Falten, und/oder zum Erhalten eines Schutz-, Korrektur- oder Restrukturierungseffekts der Haut.

17. Verfahren zur kosmetischen Pflege der Haut, welches insbesondere dazu bestimmt ist, einen Effekt der Prävention oder der Verlangsamung des Auftretens von Anzeichen der Hautalterung zu erhalten, **dadurch gekennzeichnet, dass** dieses das Aufbringen einer kosmetischen Zusammensetzung, wie in einem der Ansprüche 1 bis 15 definiert, auf mindestens einen Teil des Körpers oder des Gesichts umfasst.

## Claims

1. A cosmetic composition in the form of a serum, a lotion, an emulsion, a cream, a tinted cream, a hydrogel, a mask, a stick or a patch film, **characterized in that** it contains
- from 0.001% to 5% by dry weight of at least one extract of at least a part of an orchid of the species *Vanda teres,* in solution or as a dispersion in a cosmetically acceptable carrier compatible with topical application to the skin, and
- at least one excipient chosen from pigments, dyes, polymers, surfactants, rheology agents, fragrances, electrolytes, pH modifiers, anti-oxidants and preservatives.

2. The composition as claimed in claim 1, **characterized in that** said extract is obtained from the stem and/or the roots and/or the leaves of the orchid *Vanda teres.*

3. The composition as claimed in either of claim 1 or 2, **characterized in that** said extract is a stem extract.

4. The composition as claimed in any one of claims 1 to 3, **characterized in that** said extract is obtained by bringing at least a part of said orchid, optionally dried and/or ground, into contact with at least one polar solvent.

5. The composition as claimed in claim 4, **characterized in that** said polar solvent is chosen from the group constituted of water, C₁-C₄ alcohols, in particular ethanol, and C₂ to C₆ glycols, in particular glycerol, butylene glycol and propylene glycol.

6. The composition as claimed in claim 5, **characterized in that** said solvent is an aqueous-ethanolic mixture, preferably a mixture comprising about 90% of water for 10% of ethanol, by volume.

7. The composition as claimed in claim 4, **characterized in that** said polar solvent is a solvent in subcritical state, in particular water in subcritical state.

8. The composition as claimed in any one of claims 1 to 7, **characterized in that** said extract has been subjected to at least one decoloring and/or purifying step.

9. The composition as claimed in any one of claims 1 to 8, **characterized in that** said extract is introduced into said composition after having been absorbed onto a support, in particular onto a support comprising porous or nonporous nylon powders, or micas, or a lamellar mineral substance.

10. The composition as claimed in any one of claims 1 to 9, **characterized in that** said extract is an aqueous extract.

11. The composition as claimed in any one of claims 1 to 10, **characterized in that** it contains from 0.01% to 1% by dry weight of said extract as defined in any one of claims 1 to 8.

12. The composition as claimed in any one of claims 1 to 11, **characterized in that** it contains at least one extract of at least one other plant belonging to the orchid family (Orchidaceae).

13. The composition as claimed in any one of claims 1 to 12, **characterized in that** it also contains at least one extract of at least one other orchid of the *Vanda* genus, in particular an extract of at least a part of an orchid of the species *Vanda denisoniana* and/or of at least a part of an orchid of the species *Vanda coerulea.*

14. The composition as claimed in claim 13, **characterized in that** it contains at least one *Vanda teres* stem extract and/or one *Vanda teres* root extract, in combination with a *Vanda denisoniana* stem extract and/or a *Vanda coerulea* stem extract.

15. The composition as claimed in any one of claims 1 to 14, **characterized in that** it comprises a *Vanda teres* root extract.

16. The use, in a cosmetic composition, of an extract as defined in any one of claims 1 to 8 as a cosmetic agent for maintaining the structure of the skin, in particular by limiting the degradation of the extracellular matrix of the upper layers of the dermis and of the epidermis, and/or for reducing or delaying the effects of skin ageing, in particular the formation of wrinkles, and/or for obtaining a protective, corrective or restructuring effect on the skin.

17. A method of cosmetic care for the skin, particularly intended for obtaining an effect of prevention or slowing down of the appearance of skin ageing signs, **characterized in that** it comprises the application, to at least a part of the body or of the face, of a cosmetic composition as defined in any one of claims 1 to 15.
